# EUROPÄISCHE PATENTSCHRIFT

(11) **EP 4 162 978 B1**
(45) Veröffentlichungstag und Bekanntmachung des Hinweises auf die Patenterteilung: **16.04.2025**
(21) Anmeldenummer: 22191939.2
(22) Anmeldetag: 24.08.2022
(51) Int. Cl.: A61N 5/06

(54) **LICHTAPPLIKATOR ZUR DURCHFÜHRUNG EINER TRANSKUTANEN PHOTODYNAMISCHEN THERAPIE (PDT)**
LIGHT APPLICATOR FOR PERFORMING TRANSCUTANEOUS PHOTODYNAMIC THERAPY (PDT)
APPLICATEUR DE LUMIÈRE DESTINÉ À LA MISE EN UVRE D'UNE THÉRAPIE PHOTODYNAMIQUE TRANSCUTANÉE (PDT)

(30) Priorität: 07.10.2021 DE 102021211330
(43) Veröffentlichungstag der Anmeldung: 12.04.2023
(73) Patentinhaber: Richard Wolf GmbH, 75438 Knittlingen (DE)
(72) Erfinder: Weber, Bernd Claus, 76228 Karlsruhe (DE); Sieber, Stephan, 75438 Knittlingen-Freudenstein (DE)
(74) Vertreter: Patentanwälte Hemmer Lindfeld Frese Partnerschaft mbB

(56) Entgegenhaltungen:
- EP-A1- 3 777 974
- WO-A1-2018/197651
- WO-A1-2021/083465

## Beschreibung

Die vorliegende Offenbarung betrifft einen Lichtapplikator zur Durchführung einer transkutanen photodynamischen Therapie (PDT).

Die PDT ist eine an sich bekannte medizinische Therapie von pathologischem Gewebe eines Patienten. Dem Patienten wird dazu ein Photosensibilisator bzw. Markerstoff verabreicht, der sich selektiv an dem zu therapierenden pathologischen Gewebe anreichert. Bei der PDT wird dann Licht mittels eines Lichtapplikators oder mehrerer Lichtapplikatoren unmittelbar auf oder sogar in das pathologische Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche des Körpers, z.B. der Haut, oder einer inneren Oberfläche, z.B. der Speiseröhreninnenfläche oder Darmwandungen, angeordnet ist, dann kann das Therapielicht relativ einfach ausgekoppelt und auf die pathologische Gewebefläche gestrahlt werden. Erstreckt sich das pathologische Gewebe allerdings über ein Volumen in einem inneren Organ oder Organsegment, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischem Gewebevolumens bestrahlt wird. Dazu muss der Lichtapplikator oder die Lichtapplikatoren in das pathologische Gewebe eingestochen werden. Dies wird auch als interstitielle (durch innere Oberflächen) und/oder transkutane (durch die Haut) PDT bezeichnet. Die US 6,048,359 beschreibt beispielsweise ein System zur Durchführung einer transkutanen PDT, um ein inneres Volumen im Körper eines Patienten zu bestrahlen. Dabei wird eine Mehrzahl von nadelartigen Lichtapplikatoren parallel durch die Haut in den Körper gestochen, wobei jeder Lichtapplikator über seine Länge verteilt laterale Lichtauskopplungen aufweist, um so ein Volumen im Körper zu bestrahlen.

Nachteilig an der bekannten Lösung ist, dass die Lichtapplikatoren relativ dick ausgestaltet werden müssen und relativ komplex und teuer sind, sodass sie nicht als Einwegartikel zum einmaligen Gebrauch realisierbar sind. Außerdem ist die Form der Lichtapplikatoren nicht ergonomisch an die manuelle Handhabung angepasst, welche daher ein hohes Maß an Übung, Erfahrung und Geschick der Bedienperson erfordert. Die WO 2021/083465 A1 beschreibt einen Lichtapplikator für die PDD oder PDT in sich bewegenden Organen. Die WO 2018/197651 A1 beschreibt eine Vorrichtung zur gezielten Beleuchtung eines intraokularen Raumes eines menschlichen oder tierischen Auges. Die EP 3 777 974 A1 beschreibt einen Lichtapplikator zur Untersuchung und/oder Behandlung einer organischen Körpers.

Daraus ergibt sich die Aufgabe, einen kostengünstigeren Lichtapplikator zur Durchführung einer transkutanen PDT bereitzustellen, wobei der Lichtapplikator zum einen einfacher handhabbar und zum anderen sicherer und ggf. eng mit anderen Lichtapplikatoren zusammen einsetzbar ist.

Gemäß der vorliegenden Offenbarung wird zur Lösung dieses Problems ein Lichtapplikator bereitgestellt zur Durchführung einer transkutanen photodynamischen Therapie, PDT, in Gewebe eines organischen Körpers, wobei der Lichtapplikator
- einen sich entlang einer Einstichachse länglich erstreckenden Nadelabschnitt,
- zumindest ein lichtemittierendes Element am distalen Ende des Nadelabschnitts,
- eine sich zumindest teilweise distal vom zumindest einen lichtemittierenden Element erstreckende und zumindest teilweise lichttransparente Applikatorspitze zum Einstechen des Nadelabschnitts in das Gewebe des organischen Körpers entlang der Einstichachse, und
- ein proximal vom Nadelabschnitt angeordnetes Handgriffelement zum manuellen Positionieren des Lichtapplikators aufweist, wobei das Handgriffelement für das Positionieren und/oder Einstechen mit dem Nadelabschnitt koppelbar und für die Durchführung der PDT vom Nadelabschnitt abnehmbar ausgestaltet ist.

Das abnehmbare Handgriffelement erlaubt eine ergonomische Handhabung durch eine Bedienperson während des Positionierens und/oder Einstechens. Insbesondere beim Einstich kann unter Umständen eine relativ große manuelle Kraft durch die Bedienperson auf den möglichst dünnen Nadelabschnitt auszuüben sein. Dadurch, dass das Handgriffelement abnehmbar ausgestaltet ist, kann es groß genug ausgestaltet werden, dass es gut mit der ganzen Hand und mehreren Fingern umgriffen werden kann. Dadurch können mehrere Lichtapplikatoren zusammen eingesetzt werden und relativ eng nebeneinander eingestochen werden, ohne dass sich die Handgriffelemente gegenseitig bei der PDT blockieren. In bestimmten Ausführungsbeispielen ist es sogar möglich, ein Handgriffelement für das Positionieren und/oder Einstechen mehrerer Lichtapplikatoren zu verwenden. Außerdem übt das relativ voluminöse Handgriffelement nach Abnahme bei nicht vertikaler Einstichachse während der PDT keine lateral wirkende Gewichtskraft auf den Nadelabschnitt aus, die durch Hebelwirkung zu einer relativ großen lateralen Kraft auf die eingestochene Applikatorspitze führen kann. Solch eine laterale Kraft auf die eingestochene Applikatorspitze gilt es zur Schonung des Gewebes des Patienten und/oder der Applikatorspitze möglichst zu vermeiden bzw. zu minimieren.

Erfindungsgemäß weist der Lichtapplikator ferner ein Anschlusskabel auf zum Versorgen des Lichtapplikators mit Licht und/oder Strom mittels einer Versorgungseinheit. Das lichtemittierende Element kann passiv als eine Lichtleiterauskopplung eines Lichtleiters ausgestaltet sein, der sich der Länge nach durch den Nadelabschnitt erstreckt. Das Anschlusskabel kann dann ein Lichtleitfaserbündel aufweisen und die Versorgungseinheit Laserlicht in das daran angeschlossene Lichtleitfaserbündel einkoppeln. Vorzugsweise ist das lichtemittierende Element jedoch aktiv ausgestaltet, beispielsweise als LED, und setzt am distalen Ende des Nadelabschnitts aktiv elektrischen Strom in Licht um. Gegenüber einer Lichtleiterauskopplung hat ein aktives lichtemittierendes Element, z.B. eine LED, am distalen Ende des Nadelabschnitts den großen Vorteil, dass kein teurer Laser benötigt wird, dessen Licht mittels der Versorgungseinheit in den Lichtleiter eingekoppelt werden muss. Da die Versorgungseinheit die Lichtapplikatoren lediglich mit Strom versorgen muss, kann sie besonders einfach und günstig ausgestaltet sein. Das aktiv lichtemittierende Element weist vorzugsweise ein auf den Photosensibilisator bzw. Markerstoff abgestimmtes Leuchtspektrum auf. Alternativ oder zusätzlich kann dazu ein Lichtfilter eingesetzt werden.

Optional kann das Anschlusskabel einen proximalseitigen Anschluss zum Anschließen an einen Anschluss einer Versorgungseinheit aufweisen. Vorzugsweise weist die Versorgungseinheit eine Mehrzahl von Anschlüssen auf, um gleichzeitig eine Mehrzahl von Lichtapplikatoren anschließen und mit Strom und/oder Licht versorgen zu können.

Optional kann das Anschlusskabel einen distalseitigen Anschluss aufweisen, wobei der distalseitige Anschluss für das Positionieren und/oder Einstechen vom Nadelabschnitt abnehmbar und für die Durchführung der PDT an einen proximalseitigen Anschluss des Nadelabschnitts anschließbar ausgestaltet ist. Dies kann sinnvoll sein, um das Anschlusskabel für das Positionieren und/oder Einstechen vom Nadelabschnitt vollständig getrennt zu halten und es erst bei endgültig positioniertem und eingestochenem Nadelabschnitt über den distalseitigen Anschluss daran anzuschließen, wenn es für die PDT benötigt wird.

Optional kann der proximalseitige Anschluss des Nadelabschnitts bei angekoppeltem Handgriffelement von diesem schützend umschlossen sein und der distalseitige Anschluss des Anschlusskabels nur bei abgenommenem Handgriffelement an den proximalseitigen Anschluss des Nadelabschnitts anschließbar sein. Dadurch ist der proximalseitige Anschluss des Nadelabschnitts solange vor Beschädigung und Verunreinigung geschützt bis er für das Anschließen des Anschlusskabels benötigt wird.

Alternativ zu einem distalseitigen Anschluss des Anschlusskabels und einem dazu korrespondierenden proximalseitigen Anschluss des Nadelabschnitts kann das Anschlusskabel distalseitig fest mit dem Nadelabschnitt verbunden sein. Dies hat den großen Vorteil, dass Gewicht und Größe des proximalen Endes des Nadelabschnitts reduziert werden können, weil an dieser Stelle auf einen zusätzlichen Stecker und eine zusätzliche Buchse zur Verbindung von Anschlusskabel und Anschluss des Nadelabschnitts verzichtet werden kann. Dadurch gelingt es, die lateral mit Hebelwirkung einwirkenden Gewichtskräfte bei nicht vertikaler Einstichachse zu minimieren. Es wird einer Bedienperson auch ein weiteres Anschließen erspart, was insbesondere bei einer Vielzahl von einzusetzenden Lichtapplikatoren durchaus signifikant die Vorbereitungszeit für die PDT verkürzen kann. Auch ein fehlerhaftes Anschließen des Anschlusskabels an den Nadelabschnitt wird vermieden.

Optional kann das Handgriffelement entlang der Einstichachse eine erste Länge und das Anschlusskabel eine zweite Länge haben, wobei die zweite Länge um ein Vielfaches größer ist als die erste Länge. Ein ausreichend langes Anschlusskabel ist vorteilhaft, um möglichst viel Freiheit bei der Positionierung des Lichtapplikators bezüglich der Versorgungseinheit zu haben. Andererseits können lange Anschlusskabel zu einem Kabelgewirr und/oder Unübersichtlichkeit bei vielen parallel eingesetzten Lichtapplikatoren führen. Außerdem kann ein zu langes Anschlusskabel unhygienisch auf den Boden durchhängen oder mit anderen unsterilen Gegenständen in Kontakt kommen. Schließlich kann das Anschlusskabel durch sein Eigengewicht und/oder Zugspannung mit Hebelwirkung eine laterale Kraft auf den Nadelabschnitt ausüben, was möglichst zu minimieren ist. Daher ist ein fest mit dem Nadelabschnitt verbundenes Anschlusskabel vorzugsweise möglichst leicht und geordnet gebündelt in oder am Handgriffelement angeordnet.

Erfindungsgemäß ist das Anschlusskabel für das Positionieren und/oder Einstechen zumindest teilweise in einem Hohlraum des Handgriffelements verstaut. Gegenüber einem außerhalb vom Handgriffelement angeordnetem Kabelbündel hat ein Hohlraum im Handgriffelement zum verstauen des Anschlusskabels den Vorteil, dass das Anschlusskabel hygienisch sicher geschützt gelagert ist und die Bedienperson beim Positionieren und/oder Einstechen des Lichtapplikators nicht stört. Das Anschlusskabel ist weniger Biegungen und Knicken ausgesetzt und kann daher dünner und damit leichter ausgestaltet werden, wenn es nicht außerhalb vom Handgriffelement als Kabelbündel "baumelt".

Erfindungsgemäß weist das Handgriffelement im Hohlraum mindestens ein Verstauelement auf, um welches das Anschlusskabel geordnet gewickelt und/oder mäanderförmig angeordnet verstaut ist. Das Verstauelement kann beispielsweise eine im Hohlraum des Handgriffelements vorzugsweise entlang der Einstichachse verlaufende Spindel sein, um welche das Anschlusskabel beispielweise helixförmig aufgewickelt ist. Der Hohlraum ist im Übrigen sinnvoll, um das Gewicht des Handgriffelements möglichst gering zu halten. Das Verstauelement, wie etwa eine Spindel, kann ebenfalls nach Möglichkeit hohl ausgestaltet sein, um Gewicht zu reduzieren.

Optional kann das Anschlusskabel spiralisiert sein, d.h. derart thermisch vorbehandelt sein, dass es im entspannten Zustand eine helixartige Form einnimmt, welche auch immer wieder dann erneut eingenommen wird, wenn es vom gespannten in den entspannten Zustand übergeht. Mit dieser Maßnahme kann vermieden werden, dass das Anschlusskabel bei entsprechend kurzem Abstand zwischen Patient und Versorgungseinheit am ggf. unhygienischen Untergrund aufliegt. Vorzugsweise kann das spiralisierte Anschlusskabel helixartig auf ein sich in einem Hohlraum des Handgriffelements axial erstreckendes Verstauelement gewickelt sein.

Optional kann der proximalseitige Anschluss des Anschlusskabels in das Handgriffelement integriert sein, sodass das vom Nadelabschnitt abgenommene Handgriffelement für die Durchführung der PDT als Steckerelement zum Einstecken in den Anschluss der Versorgungseinheit dient. Vorzugsweise ist der proximalseitige Anschluss des Anschlusskabels proximalseitig im Handgriffelement integriert, sodass das Handgriffelement zum Anschließen des Anschlusskabels in axialer Richtung in den Anschluss der Versorgungseinheit einsteckbar ist. Durch Abnehmen und Entfernen des Handgriffelements vom Nadelabschnitt kann das sich im Hohlraum des Handgriffelements verstaute Anschlusskabel soweit abwickeln wie benötigt. Der Hohlraum weist vorzugsweise distalseitig eine Öffnung auf, die bei eingekoppeltem Nadelabschnitt vom proximalen Ende des Nadelabschnitts verschlossen wird. Nach Abnehmen des Handgriffelements vom Nadelabschnitt kann das Anschlusskabel durch die nunmehr geöffnete distalseitige Öffnung des Hohlraums aus dem Handgriffelement gezogen werden.

Optional kann der Lichtapplikator ferner ein Führungselement aufweisen, welches fest an einem proximalen Ende des Nadelabschnitts mit dem Nadelabschnitt verbunden oder darin integriert ist und derart zum Handgriffelement korrespondierend geformt ist, dass das Führungselement und das Handgriffelement federnd einrastend ineinander steckbar sind. Eine derartige Kopplung zwischen Nadelabschnitt und Handgriffelement ist einerseits lösbar und andererseits fest genug, um selbst bei relativ hoher manueller Krafteinwirkung auf das Handgriffelement möglichst spielfrei den Nadelabschnitt positionieren und/oder einstechen zu können. Vorzugsweise ist die Kopplung entlang der Einstichachse durch proximalwärtiges Ziehen des Handgriffelements bei festgehaltenem Führungselement lösbar. Dazu können auch Verschlusselemente vorgesehen sein, die beispielsweise durch laterales Eindrücken am Handgriffelement in eine Öffnungsposition gebracht werden können, um die Kopplung zu lösen.

Optional kann der Nadelabschnitt quer zur Einstichachse einen ersten Durchmesser und das Handgriffelement quer zur Einstichachse einen zweiten Durchmesser haben, wobei der zweite Durchmesser um ein Vielfaches größer ist als der erste Durchmesser. Die Ergonomie für das Positionieren und/oder Einstechen wird erheblich verbessert, wenn das Handgriffelement gut in der ganzen Hand liegt, um die manuelle Kraft optimal zu übertragen. Für die minimalinvasive PDT muss andererseits der Nadelabschnitt möglichst dünn und starr sein. Beispielweise kann der erste Durchmesser des Nadelabschnitts 1 mm oder weniger betragen. Der zweite Durchmesser des Handgriffelements kann hingegen 3 cm oder mehr betragen.

Optional kann das Führungselement quer zur Einstichachse einen dritten Durchmesser aufweisen, wobei der dritte Durchmesser größer als der erste Durchmesser und kleiner als der zweite Durchmesser ist. Dies ist sinnvoll, um einen sicheren Formschluss für die lösbare Kopplung zwischen Führungselement und Handgriffelement bereitzustellen, bei der das Handgriffelement das Führungselement zumindest teilweise umgreift.

Optional kann das Handgriffelement aus mindestens zwei miteinander verbundenen Teilen gefertigt sein. Wenngleich die Teile unlösbar miteinander verbunden sein können, ist dies besonders vorteilhaft für die Herstellung, um das Anschlusskabel in einem Hohlraum des Handgriffelements geordnet zu verstauen. Ein erster Teil des Handgriffelements kann in Form eines Hülsenteils eine äußere Wandung um einen Hohlraum bilden, der distalseitig und zunächst auch proximalseitig offen ist. Der zweite Teil kann ein proximalseitiger Deckelteil mit einer sich in den Hohlraum erstreckenden Spindel sein, auf welcher das Anschlusskabel aufgewickelt ist. Der Deckelteil kann dann derart mit dem Hülsenteil verschweißt oder verklebt werden, dass die proximalseitige Öffnung des Hohlraums des Hülsenteils verschlossen wird. Der Deckelteil bildet vorzugsweise den proximalseitigen Anschluss des Anschlusskabels zum Einstecken in die Versorgungseinheit.

Optional kann der Lichtapplikator ferner Elektronik zur Lichtapplikatorkennung aufweisen, wobei die Elektronik zur Lichtapplikatorkennung im Handgriffelement angeordnet ist. Dies ist besonders sinnvoll, um eine automatisch korrekte Versorgung des Lichtapplikators mittels der Versorgungseinheit sicherzustellen. Die Auswahl an möglichen Betriebsparametern kann reduziert sein oder ganz wegfallen, wenn die Versorgungseinheit den Lichtapplikator erkennt und entsprechend die auswählbaren Betriebsparameter reduziert oder sie ganz ohne Wahlmöglichkeiten vorgibt. Dies reduziert das Risiko einer fehlerhaften Einstellung von Betriebsparametern. Gerade wenn der Lichtapplikator vorzugsweise als Einwegartikel zum einmaligen Gebrauch ausgestaltet ist, kann die Lichtapplikatorkennung zu erkennen geben, ob der Lichtapplikator bereits eingesetzt wurde und entsprechend einen Betrieb gewähren oder verweigern. Auch der Einsatz von nicht autorisierten Lichtapplikatoren kann somit verweigert werden. Das Handgriffelement mit integriertem Anschluss für das Anschlusskabel bietet genug Volumen, um die Elektronik zur Lichtapplikatorkennung aufzunehmen.

Optional kann das Handgriffelement einen distalseitig verjüngten Teil aufweisen. Dies ist besonders sinnvoll, wenn eine Mehrzahl von Lichtapplikatoren besonders eng parallel zueinander positioniert werden müssen und das ergonomisch geformte Handgriffelement nicht zwischen die Lichtapplikatoren passt. Das Handgriffelement kann sich dazu tüllenartig distalwärts verjüngen, damit der distalseitig verjüngte Teil des Handgriffelements vorzugsweise ein proximales Ende des Nadelabschnitts umgreifen kann, wenn das Handgriffelement für das Positionieren und/oder Einstechen mit dem Nadelabschnitt gekoppelt ist.

Im Folgenden wird das hierin offenbarte System näher anhand der beiliegenden Figuren erläutert. Es zeigen:
- Fig. 1: eine schematische Darstellung eines Lichtapplikators zur Durchführung einer perkutanen PDT;
- Fig. 2: eine schematische Darstellung eines Lichtapplikators beim Einstechen in einen organischen Körper zur Durchführung einer perkutanen PDT;
- Fig. 3a,b: schematische Darstellungen der Problematik eines Lichtapplikators mit ergonomisch geformten Griff;
- Fig. 4a,b: schematische Darstellungen eines Beispiels einer Ausführungsform eines hierin offenbarten Lichtapplikators;
- Fig. 5a,b: schematische Darstellungen eines Beispiels einer weiteren Ausführungsform eines hierin offenbarten Lichtapplikators;
- Fig. 6a,b: schematische Darstellungen eines Beispiels einer weiteren Ausführungsform eines hierin offenbarten Lichtapplikators;
- Fig.7a,b: schematische Darstellungen eines Beispiels einer weiteren Ausführungsform eines hierin offenbarten Lichtapplikators;
- Fig. 8a,b: schematische Darstellungen eines Beispiels einer weiteren Ausführungsform eines hierin offenbarten Lichtapplikators;
- Fig. 8c-e: schematische Darstellungen eines Beispiels eines spiralisierten Anschlusskabels mit unterschiedlichen Dehnungszuständen;
- Fig. 9a,b: schematische Darstellungen eines Beispiels einer weiteren Ausführungsform eines hierin offenbarten Lichtapplikators; und
- Fig. 10a-c: schematische Darstellungen eines Beispiels einer weiteren Ausführungsform eines hierin offenbarten Lichtapplikators.

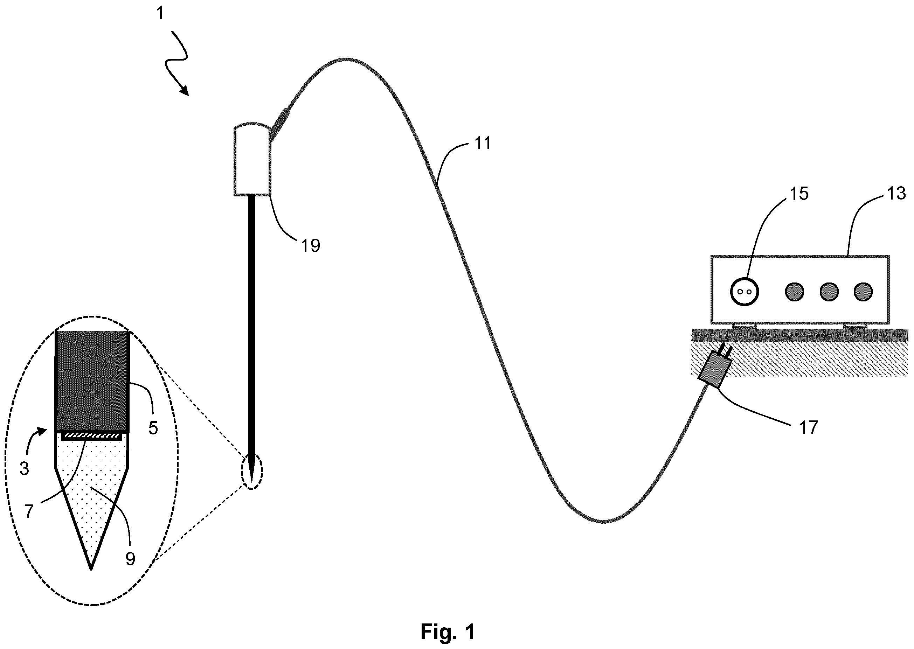

In Fig. 1 ist ein nadelartiger Lichtapplikator 1 zur Durchführung einer transkutanen photodynamischen Therapie (PDT) gezeigt, der durch die Haut eines Patienten, also transkutan, in pathologisches Gewebe des Patienten gestochen werden kann. Der Lichtapplikator 1 ist möglichst starr und dünn, um möglichst wenig, also minimalinvasiv, mit dem Einstich gesundes Gewebe des Patienten zu schädigen. Am distalen Ende 3 eines dünnen schaftförmigen Nadelabschnitts 5 des Lichtapplikators 1 ist ein aktiv lichtemittierendes Element 7 in Form einer LED angeordnet. Distalseitig von der LED 7 ist am distalen Ende 3 des Nadelabschnitts 5 des Lichtapplikators 1 eine lichttransparente und lichtstreuende Applikatorspitze 9 angeordnet. Durch die Applikatorspitze 9 wird das Licht der LED 7 möglichst isotrop in einen Raumwinkel von über 3π abgestrahlt. Die Lichtabstrahlung von der Applikatorspitze 9 ist also annähernd kugelförmig.

Der Lichtapplikator 1 ist über ein Anschlusskabel 11 an eine Versorgungseinheit 13 anschließbar, mit welcher der Lichtapplikator 1 mit Strom versorgt wird, der die LED 7 betreibt. Die Versorgungseinheit 13 weist einen Anschluss 15 für einen proximalseitigen Anschluss 17 des Anschlusskabels 11 auf. Der Lichtapplikator 1 weist am proximalen Ende des Nadelabschnitts 5 ein Handgriffelement 19 auf, mit dem eine Bedienperson den Lichtapplikator 1 manuell greifen, positionieren und in den Patienten einstechen kann. Über das Anschlusskabel 11 mit dem proximalseitigen Anschlussstecker 17, der in den Anschluss 15 der Versorgungseinheit 13 passt, kann der Lichtapplikator 1 angeschlossen und mit Strom versorgt werden. Der Nadelabschnitt 5 des Lichtapplikators 1 weist vorzugsweise einen elektrisch leitenden Kern und einen vom Kern elektrisch isolierten elektrisch leitenden Mantel auf, sodass Kern und Mantel als Hin- und Rückleiterpaar fungieren können, um die LED 7 mit Strom zu versorgen. Alternativ oder zusätzlich dazu kann ein extra Stromleiter im Nadelabschnitt 5 des Lichtapplikators 1 vorgesehen sein.

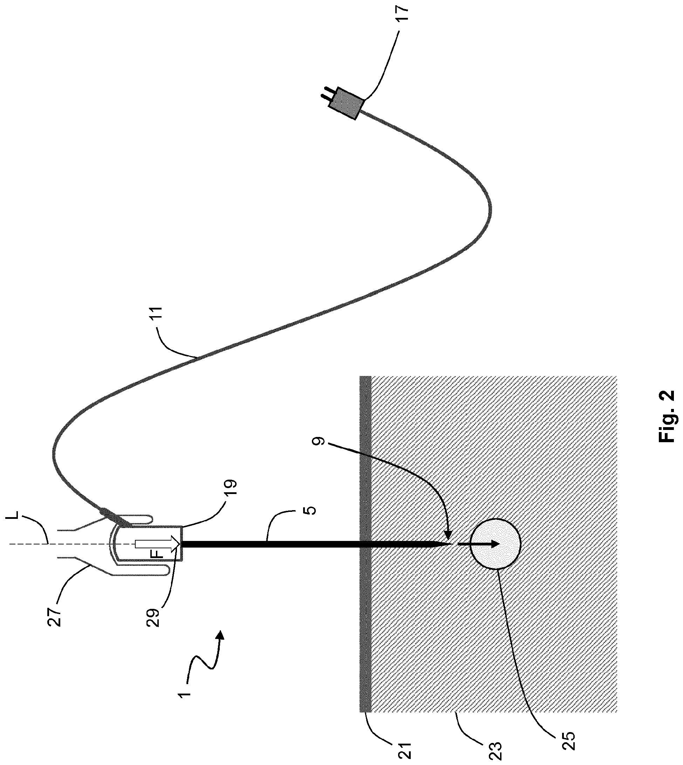

Fig. 2 zeigt den Lichtapplikator 1 beim Einstechen durch die Haut 21 eines Körpers 23 eines Patienten, mit dem Ziel, pathologisches Gewebe 25 im Patienten mit perkutaner PDT zu therapieren. Eine Bedienperson greift dazu mit ihrer Hand 27 das Handgriffelement 19 und übt eine entlang einer Einstichachse L distalwärts gerichtete Einstichkraft 29 auf den Nadelabschnitt 5 aus.

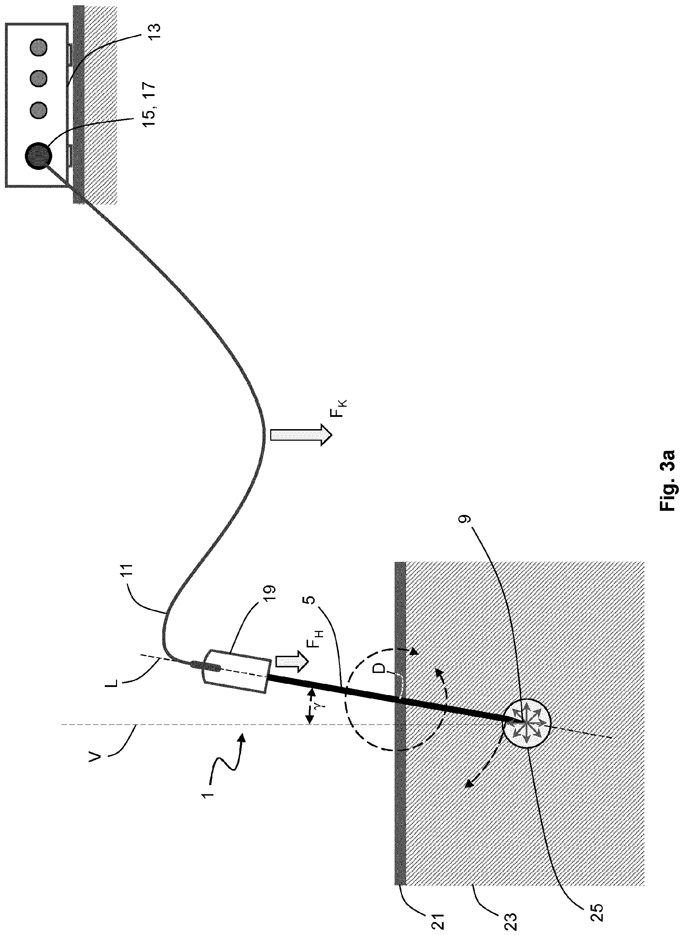

Fig. 3a zeigt eine erste Problematik von lateralen Kräften, die über Hebelkraft auf den Lichtapplikator 1 einwirken, sobald der Lichtapplikator 1 endgültig positioniert und eingestochen ist und losgelassen wird, um die PDT durchzuführen. Die Applikatorspitze 9 ist in das pathologische Gewebe 25 eingestochen, um dieses von innen heraus mit Licht zu bestrahlen. Die Einstichachse L verläuft in diesem Beispiel in einem Winkel γ zur Vertikalen V, sodass die Gewichtskraft F_{H} des Handgriffelements 19 ein Drehmoment um eine parallel zur Haut verlaufende Drehachse D auf den eingestochenen Nadelabschnitt 5 ausübt. Dieses Drehmoment um die Drehachse D führt zu einer unerwünschten gewebebelastenden lateralen Kraft auf den eingestochenen Teil des Nadelabschnitts 5. Dies ist nicht nur für das Gewebe schädlich, sondern kann im schlimmsten Fall dazu führen, dass die Applikatorspitze 9 abbricht. Die Hebelwirkung ist dabei umso größer, je kürzer der eingestochene Teil des Nadelabschnitt 5 ist gegenüber dem außerhalb des Körpers 23 befindlichen Teils des Nadelabschnitts 5. Zusätzlich zur Gewichtskraft F_{H} des Handgriffelements 19 wirkt eine Gewichtskraft F_{K} auf das an die Versorgungseinheit 13 angeschlossene Anschlusskabel 11, wenn es sich nicht unhygienisch auf einem Untergrund ablegt und wie gezeigt bis zur Versorgungseinheit 13 durch die Luft gespannt ist. Auch diese Kraft verstärkt mit relativ großer Hebelwirkung das unerwünschte Drehmoment auf den Lichtapplikator 1 um die Drehachse D.

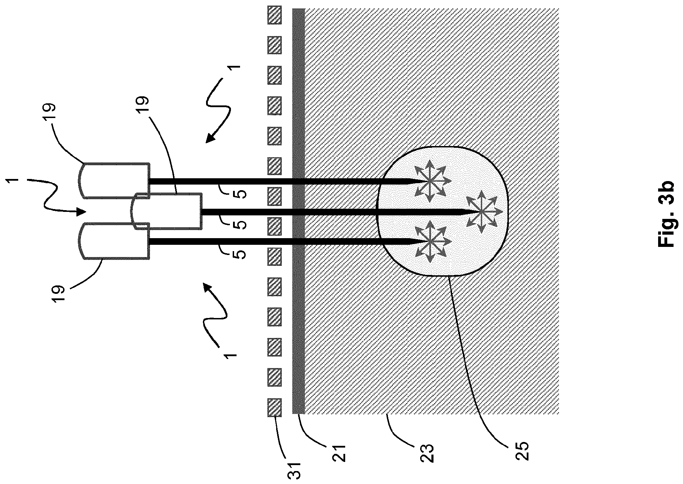

Fig. 3b zeigt eine zweite Problematik beim Einsatz mehrerer Lichtapplikatoren 1 vorzugsweise mit einer Positionierungsschablone 31, in der die Lichtapplikatoren 1 definiert geführt sind. Ist für die Durchführung der PDT in dem in diesem Fall voluminöseren pathologischen Gewebe 25 eine engmaschige PDT mit mehreren Lichtapplikatoren 1 durchzuführen, so können sich die lateral ausladenden Handgriffelemente 19 der jeweiligen Lichtapplikatoren 1 gegenseitig blockieren.

Um einerseits ein ergonomisch gut handhabbares Handgriffelement bereitzustellen und anderseits die in Fig. 3a,b gezeigten Probleme nicht oder nur eingeschränkt aufkommen zu lassen, ist in den nachfolgend beschriebenen Ausführungsbeispielen das Handgriffelement 19 abnehmbar vom Nadelabschnitt 5 ausgestaltet.

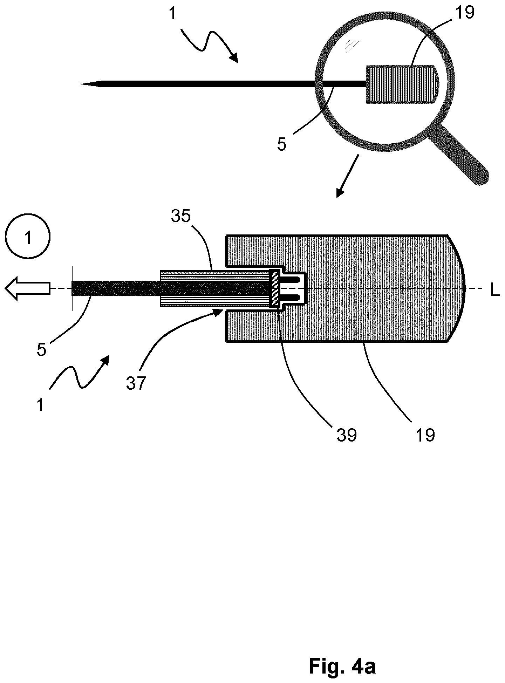

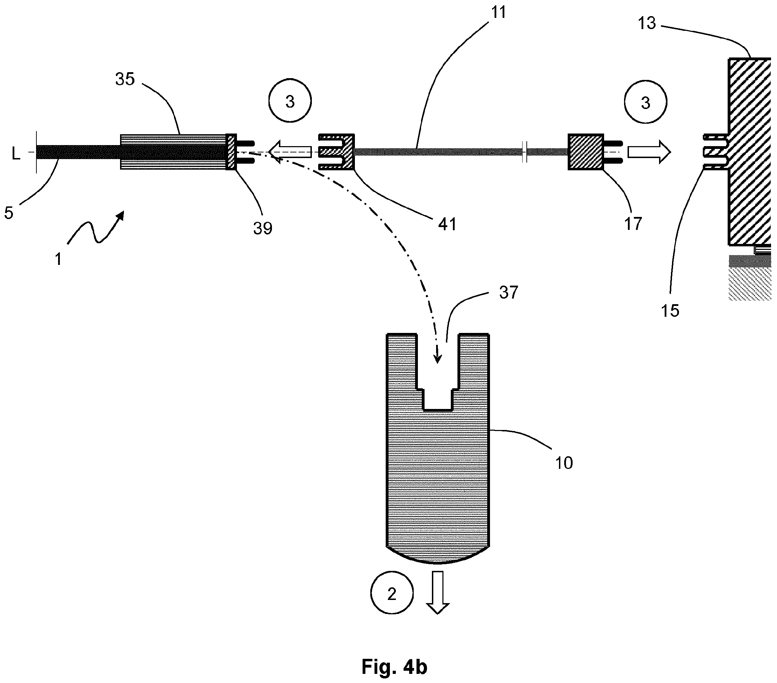

Fig. 4a zeigt schematisch einen Längsschnitt des Lichtapplikators 1, bei dem das Handgriffelement 19 koppelnd auf das proximale Ende des Nadelabschnitts 5 aufgesteckt ist. In dieser gekoppelten Konfiguration kann der Lichtapplikator manuell positioniert und eingestochen werden als wäre das Handgriffelement 19 fest mit dem Nadelabschnitt 5 verbunden. Am proximalen Ende des Nadelabschnitts 5 ist dieses etwas verdickt ausgestaltet oder mit einem Führungselement 35 in Form einer Führungshülse ausgestattet. Das Führungselement 35 ist form- und/oder kraftschlüssig in eine distalseitige Öffnung 37 des Handgriffelements 19 gesteckt. In diesem Ausführungsbeispiel weist das proximale Ende des Nadelabschnitts 5 einen proximalseitigen Anschluss 39 zum Anschließen eines distalen Anschlusses 41 (siehe Fig. 4b) des Anschlusskabels 11 auf. In der in Fig. 4a gezeigten gekoppelten Konfiguration umgibt das Handgriffelement 19 schützend den proximalseitigen Anschluss 39 des Nadelabschnitts 5, sodass das Anschlusskabels 11 erst anschließbar ist, wenn das Handgriffelements 19 vom Nadelabschnitt 5 abgenommen ist.

Sobald der Lichtapplikator 1 in einem ersten Schritt (in den Figuren durch eine eingekreiste Zahl gekennzeichnet) positioniert und/oder eingestochen ist, kann gemäß Fig. 4b in einem zweiten Schritt das Handgriffelement 19 abgenommen werden. Sodann kann ein separates Anschlusskabel 11 verwendet werden, um den Lichtapplikator 1 mit einer Versorgungseinheit 13 zu verbinden, die den Lichtapplikator 1 über das Anschlusskabel 11 mit Strom versorgt. Dazu wird der distalseitige Anschluss 41 des Anschlusskabels 11 an den proximalseitigen Anschluss 39 des Nadelabschnitt 5 angeschlossen und der proximalseitige Anschluss 17 des Anschlusskabels 11 an den Anschluss 15 der Versorgungseinheit 13 angeschlossen.

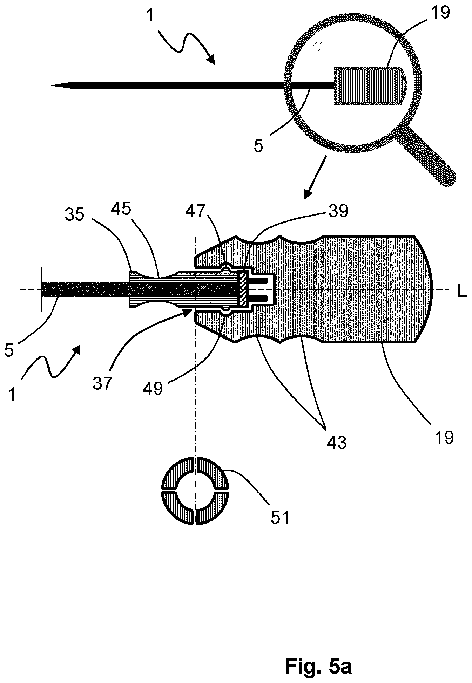

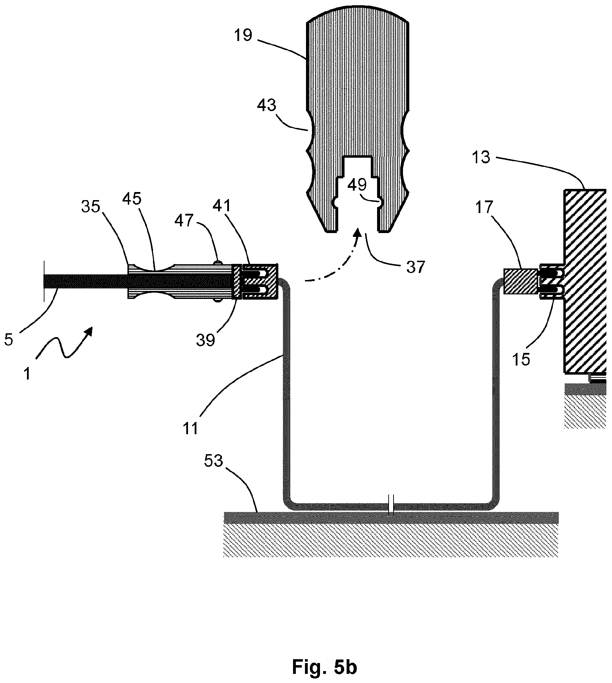

Fig. 5a zeigt das in Fig. 4a gezeigte Prinzip in einer detaillierteren Ausführungsform. Das Handgriffelement 19 weist hierbei lateral angeordnete Griffmulden 43 auf, damit das Handgriffelement 19 besser gegriffen und auf das proximale Ende des Nadelabschnitts 5 aufgesteckt oder davon abgezogen werden kann. Entsprechend weist auch das Führungselement 35 eine Griffmulde 45 auf, um es beim Koppeln und Abnehmen des Handgriffelements 19 gut festhalten zu können. Der Form- und Kraftschluss zur Kopplung wird hier durch eine außenseitige Wulst 47 am Führungselement 35 erreicht, die in eine entsprechend korrespondierend geformte innenseitige Aufnahmenut 49 im Handgriffelement 19 eingreift. Das Handgriffelement 19 ist wie im in Fig. 5a unten gezeigten Querschnitt distalseitig geschlitzt, wodurch das vorzugsweise aus Kunststoff gefertigte Handgriffelement 19 radial nach außen federnd nachgebende Federzungen 51 ausbildet. Die Federzungen 51 können von dem Wulst 47 gegen eine Federkraft nach außen gedrückt werden, um den Innendurchmesser der distalseitigen Öffnung 37 des Handgriffelements 19 kurzzeitig zu weiten, um die Wulst 41 in und aus der Aufnahmenut 49 zu bringen. Fig. 5b zeigt das nach abgenommenem Handgriffelement 19 angeschlossene Anschlusskabel 11, das sich zum Teil auf einem Untergrund 53 ablegt, wenn der Abstand zwischen Patient und dem in diesen eingeführten Lichtapplikator 1 einerseits und der Versorgungseinheit 13 andererseits entsprechend gering ist.

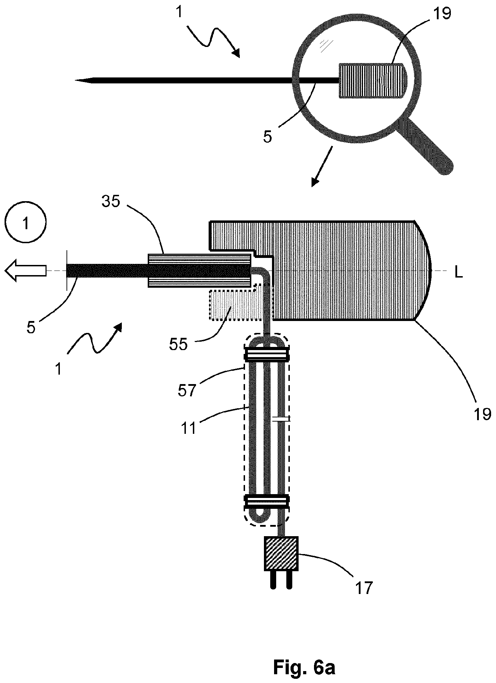

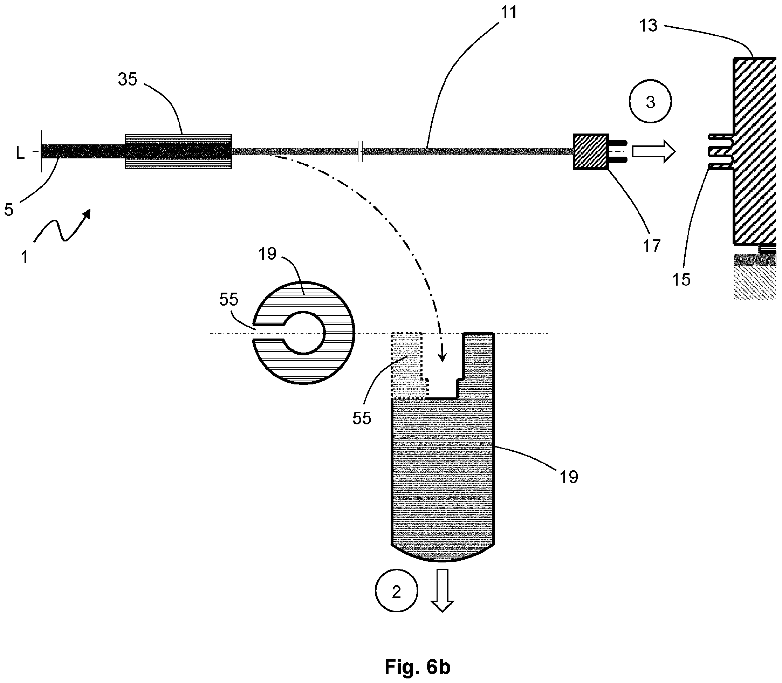

In Fig. 6a,b ist ein Ausführungsbeispiel gezeigt, bei dem das Anschlusskabel 11 fest mit dem Nadelabschnitt 5 verbunden ist. Es gibt hier also keinen distalseitigen Anschluss des Anschlusskabels 11 und keinen entsprechenden proximalseitigen Anschluss am Nadelabschnitt 5. Das Anschlusskabel 11 ist hier um etwa 90° direkt hinter dem Nadelabschnitt 11 nach außen gebogen und aus dem Handgriffelement 19 geführt. Dazu weist das Handgriffelement 19 eine distalseitig offene Kabelaussparung 55 auf.

Das Anschlusskabel 11 ist hier als Kabelbündel 57 ordentlich gebündelt, damit es die Bedienperson beim Positionieren und/oder Einstechen des Lichtapplikators 1 möglichst wenig stört. Wie in Fig. 6b gezeigt, wird das Kabelbündel 57 nach Abnahme des Handgriffelements 19 entbündelt und das Anschlusskabel 11 mit seinem proximalseitigen Anschluss 17 an den Anschluss 15 der Versorgungseinheit 13 angeschlossen.

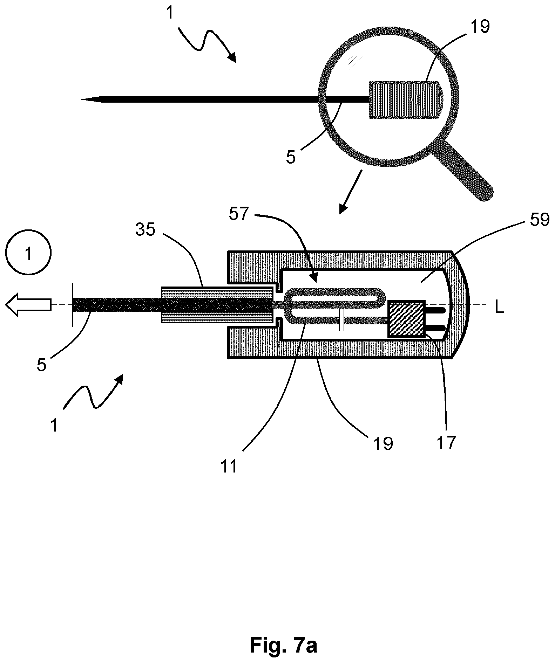

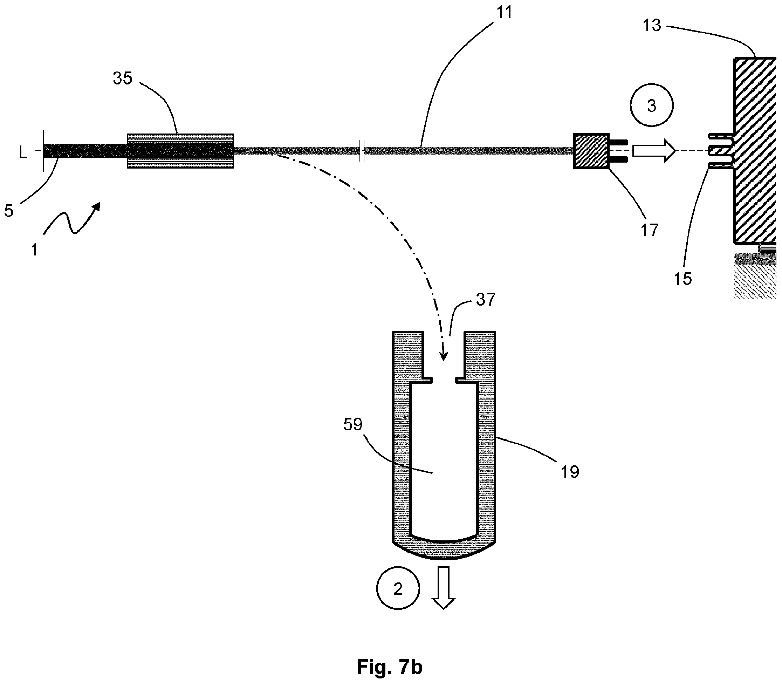

In Fig. 7a ist eine Ausführungsform gezeigt, bei der das Kabelbündel 57 samt proximalseitigem Anschluss 17 des Anschlusskabels 11 innerhalb eines Hohlraums 59 des Handgriffelements 19 verstaut ist. Dadurch stört das Kabelbündel 57 die Bedienperson beim Positionieren und/oder Einstechen des Lichtapplikators 1 überhaupt nicht. Außerdem ist das Anschlusskabel 11 so viel besser geschützt und weniger mechanischen Belastungen ausgesetzt, sodass es dünner und leichter ausgestaltet werden kann. Wie in Fig. 7b gezeigt, kann nach Abnahme des Handgriffelements 19 vom Nadelabschnitt 5 das gesamte Anschlusskabel 11 samt proximalseitigem Anschluss 17 durch die distalseitige Öffnung 37 des Handgriffelements 19 aus dem Hohlraum 59 gezogen werden, wodurch es sich entbündelt, um es an die Versorgungseinheit 13 anschließen zu können.

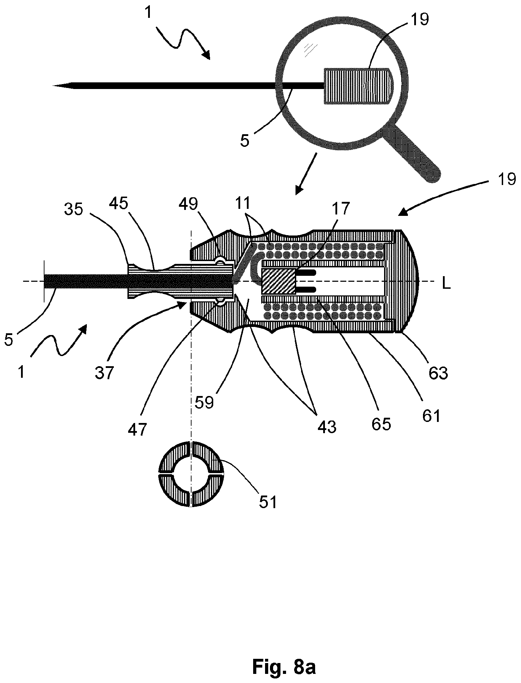

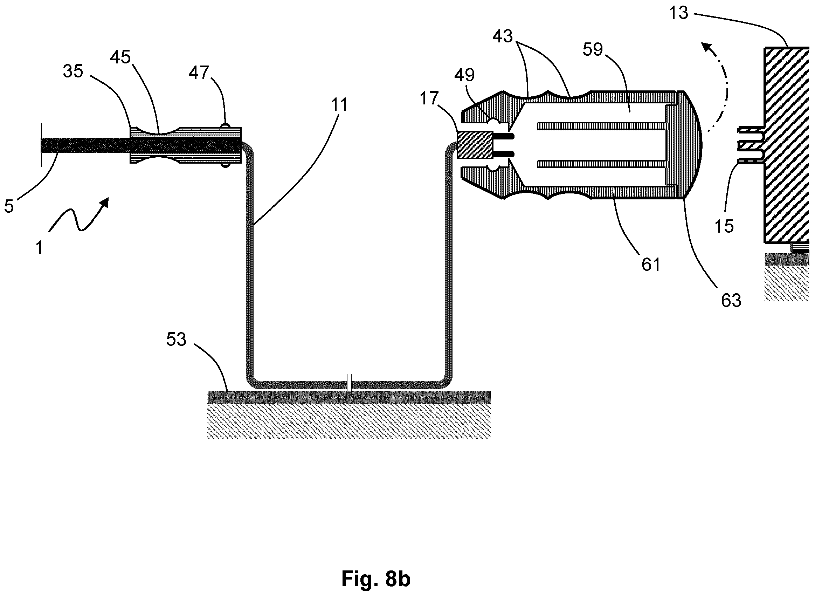

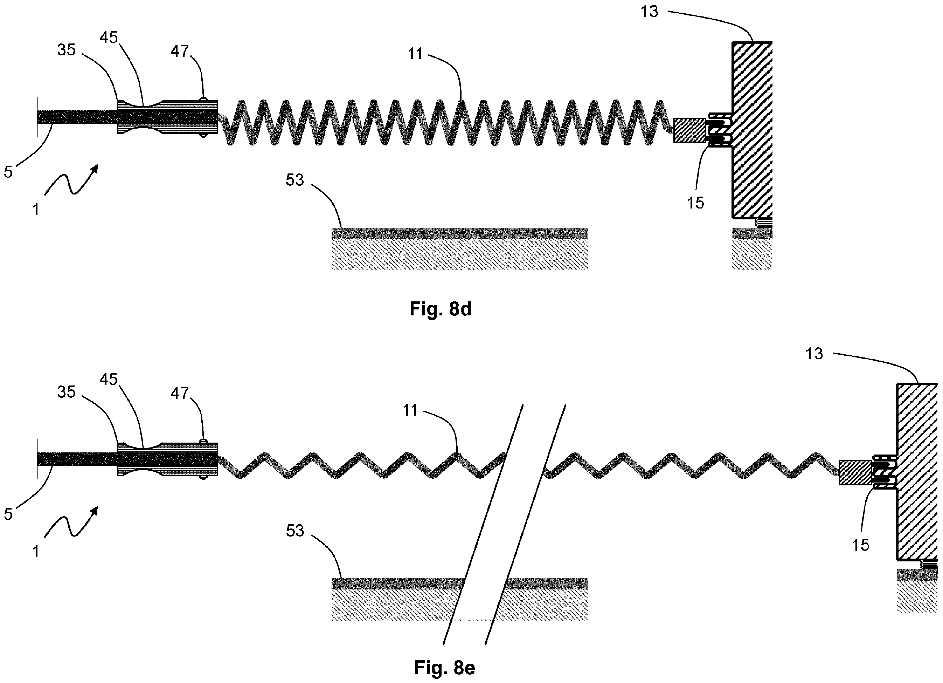

Fig. 8a,b zeigen das in Fig. 7a,b gezeigte Prinzip in einer detaillierteren Ausführungsform analog zu Fig.5a,b. Das Kopplungsprinzip und die äußere Form des Hangriffelements 19 sowie des Führungselements 35 sind wie im Ausführungsbeispiel gemäß Fig. 5a,b. Das Hangriffelement 19 ist hier allerdings aus zwei fest miteinander verbundenen Teilen 61, 63 gebildet ist. Der erste Teil 61 des Handgriffelements 19 ist hier ein Hülsenteil 61, der eine äußere Wandung um den Hohlraum 59 bildet, der distalseitig zur Öffnung 37 und vor der Verbindung mit dem zweiten Teil 63 auch proximalseitig offen ist. Der zweite Teil 63 ist hier ein proximalseitiger Deckelteil 63 mit einer sich in den Hohlraum 59 entlang der Einstichachse L erstreckenden Spindel 65, die als Verstauelement fungiert, auf welchem das Anschlusskabel 11 in der angekoppelten Konfiguration des Handgriffelements 19 aufgewickelt ist. Der Deckelteil 63 wird dann derart mit dem Hülsenteil 61 verschweißt oder verklebt, dass die proximalseitige Öffnung des Hohlraums 59 des Hülsenteils 61 verschlossen wird. Zur Gewichtsreduzierung ist die Spindel 65 hohl ausgebildet, wodurch sie den proximalseitigen Anschluss 17 des Anschlusskabels 11 platzsparend aufnehmen kann.

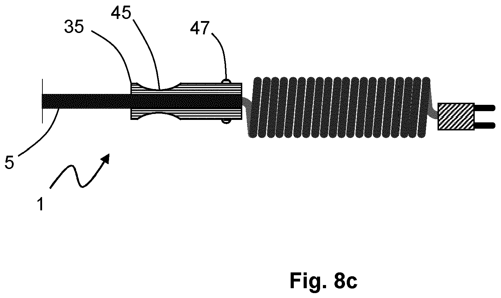

Um zu vermeiden, dass sich das Anschlusskabel 11 zum Teil auf einem ggf. unhygienischen Untergrund 53 ablegt, wenn der Abstand zwischen Patient bzw. dessen Körper 23 und dem in diesen eingeführten Lichtapplikator 1 einerseits und der Versorgungseinheit 13 andererseits entsprechend gering ist, wie das in Fig. 8b der Fall ist, kann ein Anschlusskabel 11 verwendet werden, welches dadurch gekennzeichnet ist, dass es im entspannten Zustand spiralisiert ist. Das heißt, dass das Anschlusskabel 11 dann, wenn keine Kraft auf das Anschlusskabel 11 ausgeübt wird, eine helixartige Form einnimmt, wobei die Kabelwindungen vorzugsweise direkt aneinanderliegen bzw. sich berühren und dementsprechend die axiale Ausdehnung der durch das Anschlusskabel 11 gebildeten Spirale maximal kurz ist (siehe Fig. 8c). Vorzugsweise sind in dem Zustand, wenn sich die Kabelwindungen berühren, wie in Fig. 8c dargestellt, sowohl die Durchmesser als auch die Längen der vom Anschlusskabel 11 gebildeten Spirale einerseits und der Spindel 65 andererseits aufeinander abgestimmt, so dass das spiralisierte Anschlusskabel 11 in einfacher Weise auf die Spindel 65 aufgebracht werden kann und die Spirale auch axial nicht übersteht.

Erst durch die Ausübung und die kontinuierliche Vergrößerung einer Kraft auf das Anschlusskabel 11 nimmt die axiale Ausdehnung der Spirale zu und erst dadurch wird die tatsächliche Länge des Anschlusskabels 11 mehr und mehr genutzt, was dann erforderlich wird, wenn der Abstand zwischen dem Körper 23 und der Versorgungseinheit 13 entsprechend groß ist. Die Fig.8c und d zeigen für unterschiedliche Abstände zwischen eingestochenem Lichtapplikator 1 und Versorgungseinheit 13, wie die vom Anschlusskabel 11 gebildete Spirale in axiale Richtung gedehnt wird und dadurch die eigentliche Länge des Anschlusskabels 11 situationsbezogen in unterschiedlicher Weise genutzt wird, sodass das Anschlusskabel 11 nie den Untergrund 53 berührt.

Eine solche Spiralisierung des Anschlusskabels 11 kann beispielsweise dadurch erreicht werden, dass es im aufgewickelten Zustand in einer entsprechenden Weise thermisch behandelt wird, wodurch die helixartige Form fixiert wird, sodass das Anschlusskabel 11 im entspannten Zustand diese Form aufrechterhält und dann auch beim Übergang vom gespannten in den entspannten Zustand, also dann, wenn auf das Kabel keine äußere Kraft mehr ausgeübt wird, diese Form wieder einnimmt.

Die Spiralisierung des Anschlusskabels 11 sollte dabei so durchgeführt sein, dass die zum Dehnen der Spirale in axiale Richtung erforderliche Kraft größer ist als die Gewichtskraft des Anschlusskabels 11, weil sonst die Spirale im vorliegenden Anwendungsfall bereits durch das Eigengewicht des Anschlusskabels 11 gedehnt würde und gar nicht ihre vorteilhafte kurze axiale Länge aufrechterhalten könnte.

Mit zunehmender Länge und damit zunehmendem Gewicht des Anschlusskabels 11 wird die Spiralisierung vorzugsweise derart realisiert, dass auch die auf die Kabelspirale auszuübende Kraft zur Dehnung der Kabelspirale immer größer wird. Weil aber diese Kraft auch auf den Lichtapplikator 1 als Zugkraft übertragen wird, sollte dies nicht dazu führen, dass seine Position im Körper 23 nicht mehr aufrechterhalten werden kann und ggf. ungewollt zurückgezogen und sogar ganz aus dem Körper 23 herausgezogen wird.

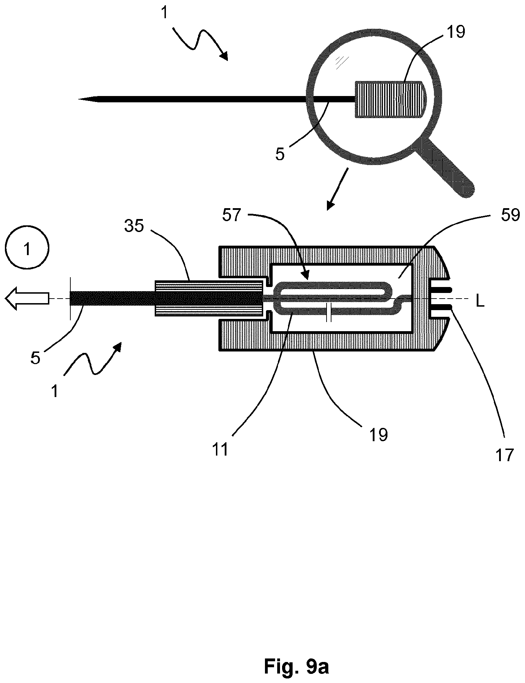

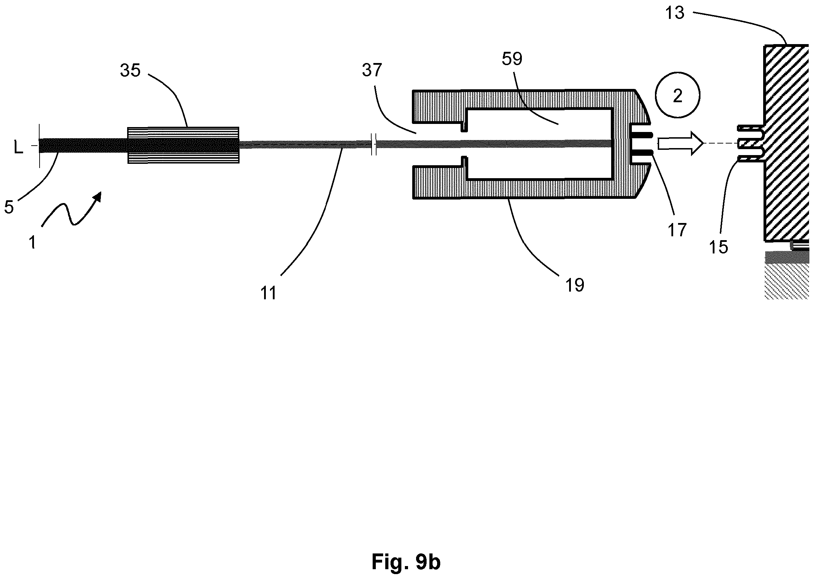

In der Ausführungsform gemäß Fig. 9a,b ist der proximalseitige Anschluss 17 des Anschlusskabels 11 in das Handgriffelement 19 integriert. Das Handgriffelement 19 bildet hier zum einen den proximalseitigen Anschluss 17 zum Einstecken in den Anschluss 15 der Versorgungseinheit 13 und zum anderen den Hohlraum 59, in dem das Anschlusskabel 11 ordentlich verstaut ist, solange das Handgriffelement 19 auf dem proximalen Ende des Nadelabschnitts 5 aufgesteckt ist. Wie in Fig. 9b gezeigt, fungiert das vom Nadelabschnitt 5 abgenommene Handgriffelement 19 als Anschlussstecker 17 für den Anschluss 15 der Versorgungseinheit 13. Mit Entfernen des Handgriffelements 19 vom Nadelabschnitt 5 entbündelt sich das Kabelbündel 57 im Hohlraum 59 und wird aus der distalseitigen Öffnung 37 des Handgriffelements 19 so weit wie benötigt herausgezogen. Falls die gesamte Länge des Anschlusskabels 11 nicht benötigt wird, kann ein ungenutzter Rest des Kabelbündels 57 gebündelt im Hohlraum 59 verbleiben, sodass sich das Anschlusskabel 11 nicht auf einem ggf. unhygienischen Untergrund 53 ablegt (wie in Fig. 5b und 8b). Außerdem ist die Kabelordnung höher, was insbesondere bei Einsatz mehrerer Lichtapplikatoren 1 gleichzeitig von Vorteil ist.

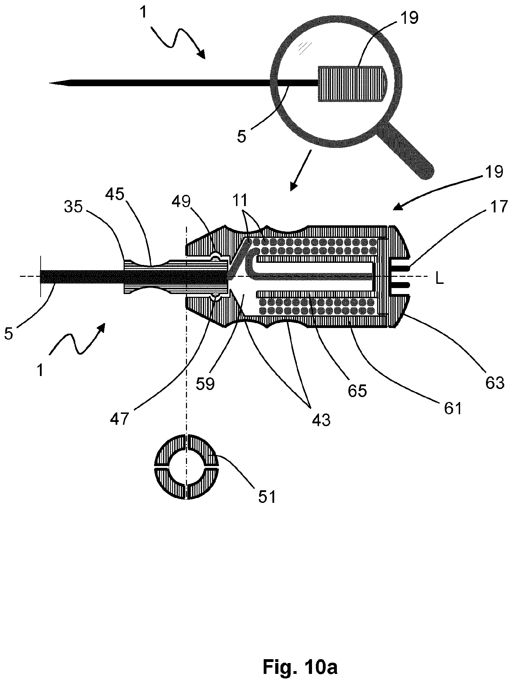

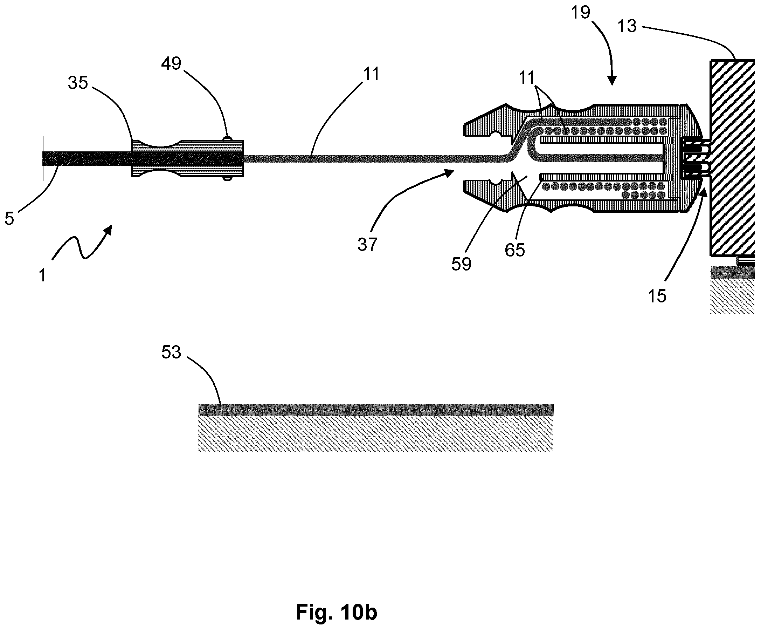

Analog zu Fig. 5a,b und 8a,b zeigen Fig. 10a-c das in Fig. 9a,b gezeigte Prinzip in einer detaillierteren Ausführungsform. Das Kopplungsprinzip und die äußere Form des Hangriffelements 19 sowie des Führungselements 35 sind wie im Ausführungsbeispiel gemäß Fig. 5a,b und Fig. 8a,b. Zusätzlich ist das Verstauen des Anschlusskabels 11 in aufgewickelter Form auf der Spindel 65 im Hohlraum 59 des Handgriffelements 19 wie im Ausführungsbeispiel gemäß Fig. 8a,b. Das in Fig. 10a-c gezeigte Ausführungsbeispiel unterscheidet sich lediglich darin vom Ausführungsbeispiel gemäß Fig. 8a,b, dass der proximalseitige Anschluss 17 des Anschlusskabels 11 in das Handgriffelement 19 integriert ist, nämlich hier im Deckelteil 63. Wie in Fig. 10b gezeigt, fungiert das vom Nadelabschnitt 5 abgenommene Handgriffelement 19 als Anschlussstecker 17 für den Anschluss 15 der Versorgungseinheit 13. Mit Entfernen des Handgriffelements 19 vom Nadelabschnitt 5 entrollt sich das auf die Spindel 65 aufgerollte Kabel 11 im Hohlraum 59 und wird aus der distalseitigen Öffnung 37 des Handgriffelements 19 nur so weit wie benötigt herausgezogen. Falls die gesamte Länge des Anschlusskabels 11 nicht benötigt wird, kann ein ungenutzter Rest des Kabels 11 auf der Spindel 65 aufgerollt im Hohlraum 59 verstaut bleiben, sodass sich das Anschlusskabel 11 nicht auf einem ggf. unhygienischen Untergrund 53 ablegt (wie in Fig. 5b und 8b). Zusätzlich kann das Anschlusskabel 11 spiralisiert sein, wie das oben beschrieben wurde. Das hat den Vorteil, dass beispielsweise auch bei einer nachträglichen Reduzierung des Abstandes zwischen Versorgungseinheit 13 und Körper 23, in welchen der Lichtapplikator 1 eingeführt ist, also dann, wenn beispielsweise die Versorgungseinheit 13 im bereits vollständig aufgerüsteten Zustand zum Körper 23 hinbewegt wird, das Anschlusskabel 11 sich nicht auf den ggf. unhygienischen Untergrund 53 ablegt, sondern sich stattdessen zu einer axial verkürzten Spirale zusammenzieht.

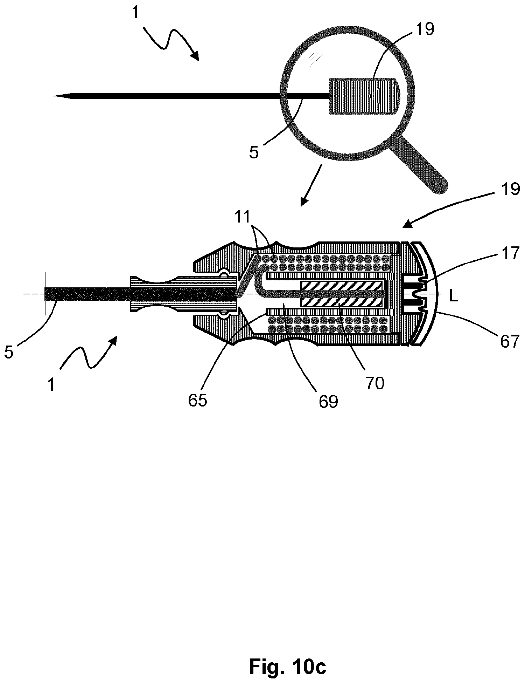

In Fig. 10c weist das Handgriffelement 19 eine Schutzkappe 67 auf, die proximalseitig den integrierten proximalseitigen Anschluss 17 des Anschlusskabels 11 schützend abdeckt, solange das Handgriffelement 19 zum Positionieren und/oder Einstechen gehalten werden muss. Die Schutzkappe 67 kann dann zum Anschließen des proximalseitigen Anschlusses 17 des Anschlusskabels 11 an den Anschluss 15 der Versorgungseinheit 13 abgenommen werden. Die Schutzkappe 67 schützt sowohl den proximalseitigen Anschluss 17 des Anschlusskabels 11 vor Verunreinigung und Beschädigung als auch die Hand 27 der Bedienperson vor scharfkantigen Pins des Anschlusses 17. Optional kann der durch die Hohlspindel 65 gebildete Hohlraum 69 Elektronikkomponenten 70, beispielsweise Elektronikkomponenten 70 zur Lichtleiterkennung, aufnehmen.

### Bezugszeichenliste:

- 1: Lichtapplikator
- 3: distales Ende des Nadelabschnitts
- 5: Nadelabschnitt
- 7: lichtemittierendes Element / LED
- 9: Applikatorspitze
- 11: Anschlusskabel
- 13: Versorgungseinheit
- 15: Anschluss der Versorgungseinheit
- 17: proximalseitiger Anschluss des Anschlusskabels
- 19: Handgriffelement
- 21: Haut
- 23: Körper
- 25: pathologisches Gewebe
- 27: Hand einer Bedienperson
- 29: Einstichkraft
- 31: Positionierungsschablone
- 33: Führungen der Positionierungsschablone
- 35: Führungselement
- 37: distalseitige Öffnung des Handgriffelements
- 39: proximalseitiger Anschluss des Nadelabschnitt
- 41: distalseitiger Anschluss des Anschlusskabels
- 43: Griffmulden
- 45: Griffmulde
- 47: Wulst
- 49: Aufnahmenut
- 51: Federzungen
- 53: Untergrund
- 55: Kabelaussparung
- 57: Kabelbündel
- 59: Hohlraum
- 61: Hülsenteil des Handgriffelements
- 63: Deckelteil des Handgriffs
- 65: Verstauelement / Spindel
- 67: Schutzkappe
- 69: Hohlraum
- 70: Elektronikkomponenten
- L: Einstichachse
- V: Vertikale
- γ: Winkel

## Patentansprüche

1. Lichtapplikator (1) zur Durchführung einer transkutanen photodynamischen Therapie, PDT, in Gewebe (25) eines organischen Körpers (23), wobei der Lichtapplikator (1)
- einen sich entlang einer Einstichachse (L) länglich erstreckenden Nadelabschnitt (5),
- zumindest ein lichtemittierendes Element (7) am distalen Ende (3) des Nadelabschnitts (5),
- eine sich zumindest teilweise distal vom zumindest einen lichtemittierenden Element (7) erstreckende und zumindest teilweise lichttransparente Applikatorspitze (9) zum Einstechen des Nadelabschnitts (5) in das Gewebe (25) des organischen Körpers (23) entlang der Einstichachse (L),
- ein Anschlusskabel (11) zum Versorgen des Lichtapplikators (1) mit Licht und/oder Strom mittels einer Versorgungseinheit (13), und
- ein proximal vom Nadelabschnitt (5) angeordnetes Handgriffelement (19) zum manuellen Positionieren des Lichtapplikators (1) aufweist,
wobei das Handgriffelement (19) einen Hohlraum (59) aufweist, und
wobei das Handgriffelement (19) für das Positionieren und/oder Einstechen mit dem Nadelabschnitt (5) koppelbar und für die Durchführung der PDT vom Nadelabschnitt (5) abnehmbar ausgestaltet ist, wobei das Anschlusskabel (11) für das Positionieren und/oder Einstechen zumindest teilweise im Hohlraum (59) des Handgriffelements (19) verstaubar ist, wobei das Handgriffelement (19) im Hohlraum (59) mindestens ein Verstauelement (65) aufweist, um welches das Anschlusskabel (11) geordnet gewickelt und/oder mäanderförmig angeordnet verstaubar ist.

2. Lichtapplikator (1) nach Anspruch 1, wobei das Anschlusskabel (11) einen proximalseitigen Anschluss (17) zum Anschließen an einen Anschluss (15) der Versorgungseinheit (13) aufweist.

3. Lichtapplikator (1) nach Anspruch 1 oder 2, wobei das Anschlusskabel (11) einen distalseitigen Anschluss (41) aufweist, wobei der distalseitige Anschluss (41) für das Positionieren und/oder Einstechen vom Nadelabschnitt (5) abnehmbar und für die Durchführung der PDT an einen proximalseitigen Anschluss (39) des Nadelabschnitts (5) anschließbar ausgestaltet ist.

4. Lichtapplikator (1) nach Anspruch 3, wobei der proximalseitige Anschluss (39) des Nadelabschnitts (5) bei angekoppeltem Handgriffelement (19) von diesem schützend umschlossen ist und der distalseitige Anschluss (41) des Anschlusskabels (11) nur bei abgenommenem Handgriffelement (19) an den proximalseitigen Anschluss (39) des Nadelabschnitts (5) anschließbar ist.

5. Lichtapplikator (1) nach Anspruch 1 oder 2, wobei das Anschlusskabel (11) distalseitig fest mit dem Nadelabschnitt (5) verbunden ist.

6. Lichtapplikator (1) nach einem der vorhergehenden Ansprüche, wobei das Handgriffelement (19) entlang der Einstichachse (L) eine erste Länge hat und das Anschlusskabel (11) eine zweite Länge (L) hat, wobei die zweite Länge um ein Vielfaches größer ist als die erste Länge.

7. Lichtapplikator (1) nach einem der vorhergehenden Ansprüche, wobei das Anschlusskabel (11) im entspannten Zustand spiralisiert ist.

8. Lichtapplikator (1) nach vorhergehenden Ansprüche, wobei das Anschlusskabel (11) helixartig auf das Verstauelement (65) gewickelt ist.

9. Lichtapplikator (1) nach einem der Ansprüche 2 bis 8, wobei der proximalseitige Anschluss (17) des Anschlusskabels (11) in das Handgriffelement (19) integriert ist, sodass das vom Nadelabschnitt (5) abgenommene Handgriffelement (19) für die Durchführung der PDT als Steckerelement zum Einstecken in den Anschluss (15) der Versorgungseinheit (13) dient.

10. Lichtapplikator (1) nach einem der vorhergehenden Ansprüche, ferner aufweisend ein Führungselement (35), welches fest an einem proximalen Ende des Nadelabschnitts (5) mit dem Nadelabschnitt (5) verbunden oder darin integriert ist und derart zum Handgriffelement (19) korrespondierend geformt ist, dass das Führungselement (35) und das Handgriffelement (19) federnd einrastend ineinander steckbar sind.

11. Lichtapplikator (1) nach einem der vorhergehenden Ansprüche, wobei der Nadelabschnitt (5) quer zur Einstichachse (L) einen ersten Durchmesser hat und das Handgriffelement (19) quer zur Einstichachse (L) einen zweiten Durchmesser hat, wobei der zweite Durchmesser um ein Vielfaches größer ist als der erste Durchmesser.

12. Lichtapplikator (1) nach Anspruch 10 und 11, wobei das Führungselement (35) quer zur Einstichachse (L) einen dritten Durchmesser aufweist, wobei der dritte Durchmesser größer als der erste Durchmesser und kleiner als der zweite Durchmesser ist.

13. Lichtapplikator (1) nach einem der vorhergehenden Ansprüche, wobei das Handgriffelement (19) aus mindestens zwei miteinander verbundenen Teilen (61, 63) gefertigt ist.

14. Lichtapplikator (1) nach einem der vorhergehenden Ansprüche, ferner aufweisend Elektronik zur Lichtapplikatorkennung, wobei die Elektronik zur Lichtapplikatorkennung im Handgriffelement (19) angeordnet ist.

15. Lichtapplikator (1) nach einem der vorhergehenden Ansprüche, wobei das Handgriffelement (19) einen distalseitig verjüngten Teil aufweist.

16. Lichtapplikator (1) nach Anspruch 15, wobei der distalseitig verjüngte Teil des Handgriffelements (19) dazu ausgestaltet ist, ein proximales Ende des Nadelabschnitts (5) zu umgreifen, wenn das Handgriffelement (19) für das Positionieren und/oder Einstechen mit dem Nadelabschnitt (5) gekoppelt ist.

## Claims

1. Light applicator (1) for the execution of a transcutaneous photodynamic therapy, PDT, in tissue (25) of an organic body (23), wherein the light applicator (1) has:
- a needle section (5) extending longitudinally along an axis of penetration (L),
- at least one light-emitting element (7) at the distal end (3) of the needle section (5),
- an applicator tip (9) extending, at least partially, distally from the at least one light-emitting element (7), and, at least partially, transparent to light for the penetration of the needle section (5) into the tissue (25) of the organic body (23) along the axis of penetration (L),
- a connection cable (11) for the supply of the light applicator (1) with light and/or power by means of a supply unit (13), and
- a handgrip element (19), arranged in the proximal direction from the needle section (5), for the manual positioning of the light applicator (1), wherein
the handgrip element (19) has a cavity (59), and wherein
the handgrip element (19) can be coupled to the needle section (5) for the positioning and/or penetration, and for the execution of the PDT is designed to be removable from the needle section (5), wherein
for the positioning and/or penetration, the connection cable (11) is, at least partially, stowable in the cavity (59) of the handgrip element (19), wherein
the handgrip element (19) has at least one stowage element (65) in the cavity (59), around which the connection cable (11) can be stowed, wound in an orderly manner, and/or in a meandering arrangement.

2. Light applicator (1) in accordance with Claim 1, wherein the connection cable (11) has a connector (17) on the proximal side for purposes of connecting to a terminal (15) of the supply unit (13).

3. Light applicator (1) in accordance with Claim 1 or 2, wherein the connection cable (11) has a connector (41) on the distal side, wherein the distal-side connector (41) can be removed from the needle section (5) for the positioning and/or penetration, and can be connected to a proximal-side terminal (39) of the needle section (5) for purposes of performing the PDT.

4. Light applicator (1) in accordance with Claim 3, wherein the proximal-side terminal (39) of the needle section (5) is protectively enclosed by the handgrip element (19) when the latter is coupled, and the distal-side connector (41) of the connection cable (11) can only be connected to the proximal-side terminal (39) of the needle section (5) when the handgrip element (19) is removed.

5. Light applicator (1) in accordance with Claim 1 or 2, wherein on the distal side the connection cable (11) is fixedly connected to the needle section (5).

6. Light applicator (1) in accordance with one of the preceding claims, wherein the handgrip element (19) has a first length along the axis of penetration (L), and the connection cable (11) has a second length (L), wherein the second length is multiple times greater than the first length.

7. Light applicator (1) in accordance with one of the preceding claims, wherein in the relaxed state the connection cable (11) has the form of a spiral.

8. Light applicator (1) in accordance with preceding claims, wherein the connection cable (11) is wound helically onto the stowage element (65).

9. Light applicator (1) in accordance with one of the Claims 2 to 8, wherein the proximal-side connector (17) of the connection cable (11) is integrated into the handgrip element (19), such that the handgrip element (19), removed from the needle section (5), serves as a plug-in element for insertion into the terminal (15) of the supply unit (13) for the execution of the PDT.

10. Light applicator (1) in accordance with one of the preceding claims, further comprising a guide element (35), which is fixedly connected to the needle section (5) at a proximal end of the needle section (5), or is integrated therein, and is shaped to correspond to the handgrip element (19) such that the guide element (35) and the handgrip element (19) can be inserted into one another in a spring-loaded manner.

11. Light applicator (1) in accordance with one of the preceding claims, wherein the needle section (5) has a first diameter transverse to the axis of penetration (L), and the handgrip element (19) has a second diameter transverse to the axis of penetration (L), wherein the second diameter is multiple times greater than the first diameter.

12. Light applicator (1) in accordance with Claims 10 and 11, wherein the guide element (35) has a third diameter transverse to the axis of penetration (L), wherein the third diameter is greater than the first diameter, and less than the second diameter.

13. Light applicator (1) in accordance with one of the preceding claims, wherein the handgrip element (19) is fabricated from at least two interconnected parts (61, 63).

14. Light applicator (1) in accordance with one of the preceding claims, further comprising electronics for purposes of light applicator identification, wherein the electronics for purposes of light applicator identification are arranged in the handgrip element (19).

15. Light applicator (1) in accordance with one of the preceding claims, wherein the handgrip element (19) comprises a part that is tapered on the distal side.

16. Light applicator (1) in accordance with Claim 15, wherein the part of the handgrip element (19) that is tapered on the distal side is designed to grip around a proximal end of the needle section (5), when the handgrip element (19) is coupled to the needle section (5) for the positioning and/or penetration.

## Revendications

1. Applicateur de lumière (1) pour exécuter un traitement photodynamique transcutané, PDT, dans le tissu (25) d'un corps organique (23), dans lequel l'applicateur de lumière (1) présente - un tronçon d'aiguille (5) s'étendant longitudinalement le long d'un axe de piquage (L),
- au moins un élément émetteur de lumière (7) à l'extrémité distale (3) du tronçon d'aiguille (5),
- une pointe d'applicateur (9) s'étendant au moins partiellement de manière distale de l'au moins un élément émetteur de lumière (7) et au moins partiellement transparent à la lumière pour piquer le tronçon d'aiguille (5) dans le tissu (25) du corps organique (23) le long de l'axe de piquage (L),
- un câble de raccordement (11) pour alimenter l'applicateur de lumière (1) en lumière et/ou en courant électrique au moyen d'une unité d'alimentation (13), et
- un élément de poignée (19) disposé de manière proximale au tronçon d'aiguille (5) pour le positionnement manuel de l'applicateur de lumière (1),
dans lequel l'élément de poignée (19) présente un espace creux (59), et
dans lequel l'élément de poignée (19) est conçu en pouvant être couplé au tronçon d'aiguille (5) pour le positionnement et/ou le piquage et en pouvant être retiré du tronçon d'aiguille (5) pour exécuter le PDT, dans lequel le câble de raccordement (11) peut être au moins partiellement rangé dans la cavité (59) de l'élément de poignée (19) pour le positionnement et/ou le piquage, dans lequel l'élément de poignée (19) présente dans la cavité (59) au moins un élément de rangement (65) autour duquel le câble de raccordement (11) peut être rangé en étant disposé enroulé et/ou en formant des sinuosités.

2. Applicateur de lumière (1) selon la revendication 1, dans lequel le câble de raccordement (11) présente un raccord (17) côté proximal pour le raccordement à un raccord (15) de l'unité d'alimentation (13).

3. Applicateur de lumière (1) selon la revendication 1 ou 2, dans lequel le câble de raccordement (11) présente un raccord côté distal (41), dans lequel le raccord côté distal (41) est conçu en pouvant être retiré du tronçon d'aiguille (5) pour le positionnement et/ou le piquage et en pouvant être raccordé à un raccord côté proximal (39) du tronçon d'aiguille (5) pour exécuter le PDT.

4. Applicateur de lumière (1) selon la revendication 3, dans lequel le raccord (39) côté proximal du tronçon d'aiguille (5) est entouré de manière protectrice par l'élément de poignée (19) lorsque celui-ci est couplé, et le raccord côté distal (41) du câble de raccordement (11) peut être raccordé au raccord (39) côté proximal du tronçon d'aiguille (5) uniquement lorsque l'élément de poignée (19) est retiré.

5. Applicateur de lumière (1) selon la revendication 1 ou 2, dans lequel le câble de raccordement (11) est raccordé fixement au tronçon d'aiguille (5) côté distal.

6. Applicateur de lumière (1) selon l'une des revendications précédentes, dans lequel l'élément de poignée (19) a une première longueur le long de l'axe de piquage (L), et le câble de raccordement (11) présente une seconde longueur (L), dans lequel la seconde longueur est beaucoup plus grande que la première longueur.

7. Applicateur de lumière (1) selon l'une des revendications précédentes, dans lequel le câble de raccordement (11) est en spirale dans l'état détendu.

8. Applicateur de lumière (1) selon l'une des revendications précédentes, dans lequel le câble de raccordement (11) est enroulé à la manière d'une hélice sur l'élément de rangement (65).

9. Applicateur de lumière (1) selon l'une des revendications 2 à 8, dans lequel le raccord (17) côté proximal du câble de raccordement (11) est intégré dans l'élément de poignée (19), de sorte que l'élément de poignée (19) retiré du tronçon d'aiguille (5) pour l'exécution du PDT sert d'élément de connecteur pour la connexion dans le raccord (15) de l'unité d'alimentation (13).

10. Applicateur de lumière (1) selon l'une des revendications précédentes, présentant en outre un élément de guidage (35) qui est relié fixement au tronçon d'aiguille (5) à une extrémité proximale du tronçon d'aiguille (5) ou intégré à l'intérieur et est formé en correspondance avec l'élément de poignée (19) de telle sorte que l'élément de guidage (35) et l'élément de poignée (19) peuvent être connectés l'un dans l'autre par encliquetage de manière élastique.

11. Applicateur de lumière (1) selon l'une des revendications précédentes, dans lequel le tronçon d'aiguille (5) présente un premier diamètre transversalement à l'axe de piquage (L) et l'élément de poignée (19) présente un deuxième diamètre transversalement à l'axe de piquage (L), dans lequel le deuxième diamètre est beaucoup plus grand que le premier diamètre.

12. Applicateur de lumière (1) selon la revendication 10 et 11, dans lequel l'élément de guidage (35) présente un troisième diamètre transversalement à l'axe de piquage (L), dans lequel le troisième diamètre est plus grand que le premier diamètre et plus petit que le deuxième diamètre.

13. Applicateur de lumière (1) selon l'une des revendications précédentes, dans lequel l'élément de poignée (19) est fabriqué à partir d'au moins deux parties (61, 63) reliées entre elles.

14. Applicateur de lumière (1) selon l'une des revendications précédentes, présentant en outre une électronique pour l'identification de l'applicateur de lumière, dans lequel l'électronique pour l'identification de l'applicateur de lumière est disposée dans l'élément de poignée (19).

15. Applicateur de lumière (1) selon l'une des revendications précédentes, dans lequel l'élément de poignée (19) présente une partie rétrécie côté distal.

16. Applicateur de lumière (1) selon la revendication 15, dans lequel la partie rétrécie côté distal de l'élément de poignée (19) est conçue pour entourer une extrémité proximale du tronçon d'aiguille (5), lorsque l'élément de poignée (19) est couplé au tronçon d'aiguille (5) pour le positionnement et/ou le couplage.
